# EUROPEAN PATENT APPLICATION

(11) **EP 3 912 659 A1**
(43) Date of publication of application: **24.11.2021**
(21) Application number: 20740891.5
(22) Date of filing: 17.01.2020
(51) Int. Cl.: A61M 5/175, A61M 5/152, A61M 5/168

(54) **DRUG SOLUTION INJECTION CONTROLLER**

(30) Priority: 17.01.2019 JP 2019006387
(71) Applicant: Nipro Corporation, Osaka-shi, Osaka 531-8510 (JP)
(72) Inventor: SAKAMOTO, Shingo, Osaka-shi, Osaka 531-8510 (JP)
(74) Representative: Slingsby Partners LLP
(86) International application number: PCT/JP2020/001543
(87) International publication number: WO 2020/149406

(57) **Abstract**

A pressing operation member 78 for performing rapid administration of a drug solution is attached, so as to be movable back and forth, to a housing 34 that houses a sub-reservoir 36. Arranged side by side in series in a direction of movement of the pressing operation member 78 are a pressing operation surface 79 of the pressing operation member 78, the sub-reservoir 36, and a flow path arrangement space 115 for inflow side and outflow side flow paths of the drug solution with respect to the sub-reservoir 36. Disposed in the flow path arrangement space 115 are a rapid administration valve 116 for opening and closing an outflow side flow path 96, a limiting flow path 94 provided on an inflow side flow path 92, a priming flow path 95 bypassing the limiting flow path 94, and a closing valve 140 for closing the priming flow path 95.

## Description

### TECHNICAL FIELD

The present invention relates to a drug solution injection controller for performing rapid injection of a drug solution by self-operation in a drug solution administration device for continuous administration of the drug solution.

### BACKGROUND ART

Conventionally, a drug solution administration device that continuously administers a drug solution from a main reservoir through a main line has been known. The drug solution administration device is used, for example, when continuously administering painkillers, anesthetics, etc. into the body little by little. A flow rate control par for limiting the flow rate of the drug solution is provided to the main line connecting the main reservoir and the connector on the drug solution input side to the patient.

Meanwhile, the drug solution administration device may include a drug solution injection controller for rapid administration of the drug solution by self-operation. The drug solution injection controller is provided with a sub-reservoir for storing the drug solution. For example, by the patient performing a self-operation such as pressing a push button, the drug solution stored in the sub-reservoir of the drug solution injection controller will be rapidly administered into the patient's body through a sub-line branched from the main line. The sub-line is a line including an inflow side flow path and an outflow side flow path of the drug solution with respect to the sub-reservoir.

The drug solution injection controller suffers from a problem that, on the one hand, in order to prevent overdose of the drug solution due to rapid administrations of the drug solution at short intervals, high functionality such as a limiting flow path that limits the flow rate on the flow path that supplies the drug solution to the sub-reservoir is required, but on the other hand, the higher the function, the larger the size becomes.

### SUMMARY OF THE INVENTION

### PROBLEM THE INVENTION ATTEMPTS TO SOLVE

The present invention has been developed in view of the above-described matters as the background, and it is an object of the present invention to provide a drug solution injection controller with a novel structure which is able to quickly complete the priming of the sub-line including the inflow side flow path and the outflow side flow path of the drug solution with respect to the sub-reservoir while being compact.

### MEANS FOR SOLVING THE PROBLEM

The above and/or optional objects of this invention may be attained according to at least one of the following preferred embodiments of the invention. The following preferred embodiments and/or elements employed in each preferred embodiment of the invention may be adopted at any possible optional combinations.

That is, the first preferred embodiment of the present invention provides a drug solution injection controller comprising: a housing; a sub-reservoir housed by the housing; and a pressing operation member for performing rapid administration of a drug solution, the pressing operation member being attached to the housing so as to be movable back and forth, wherein a pressing operation surface of the pressing operation member, the sub-reservoir, and a flow path arrangement space in which an inflow side flow path and an outflow side flow path of the drug solution with respect to the sub-reservoir are arranged are provided side by side in series in a direction of movement of the pressing operation member, and the flow path arrangement space is provided with a rapid administration valve for opening and closing the outflow side flow path, a limiting flow path provided on the inflow side flow path, a priming flow path bypassing the limiting flow path on the inflow side flow path, and a closing valve for closing the priming flow path.

According to the drug solution injection controller structured following the present preferred embodiment, the flow path arrangement space is provided in the direction of movement of the pressing operation member with respect to the sub-reservoir, and in the flow path arrangement space, the priming flow path bypassing the limiting flow path is provided, and the closing valve for closing the priming flow path is provided. By injecting the priming liquid in the open state of the priming flow path, the priming liquid quickly fills the sub-reservoir and the sub-line including the inflow side flow path and the outflow side flow path of the drug solution with respect to the sub-reservoir through the priming flow path. Therefore, the time required for priming the sub-line can be shortened.

Further, by closing the priming flow path by the closing valve after the priming of the sub-line is completed, the time required for refilling the sub reservoir with the drug solution in is set by the limiting flow path. Therefore, it is possible to prevent continuous rapid administration of the drug solution, thereby preventing overdose of the drug solution.

Furthermore, the pressing operation surface of the pressing operation member, the sub-reservoir, and the flow path arrangement space in which the inflow side flow path and the outflow side flow path of the drug solution with respect to the sub-reservoir are arranged are provided side by side in series in the direction of moving back and forth of the pressing operation member. Accordingly, the drug solution injection controller can have a compact structure in the direction orthogonal to the direction of moving back and forth of the pressing operation member. Therefore, for example, when the user pushes the pressing operation surface with a thumb while grasping the housing with one hand, the user can easily grasp the housing, the pressing operation of the pressing operation surface becomes easy, and the drug solution injection controller is less likely to be bulky when stored in the user's pocket or the like.

The second preferred embodiment of the present invention provides the drug solution injection controller according to the first preferred embodiment, wherein the sub-reservoir is arranged closer to the pressing operation member than a center of the housing in the direction of movement of the pressing operation member.

According to the drug solution injection controller structured following the present preferred embodiment, it is possible to secure a large space for arranging the inflow side flow path and the outflow side flow path, which are provided side by side in series with the sub-reservoir, without increasing the size of the housing.

A third preferred embodiment of the present invention provides the drug solution injection controller according to the first or second preferred embodiment, further comprising: a plunger for pressing the sub-reservoir during pressing operation of the pressing operation member; and a coil spring urging the plunger toward the sub-reservoir, wherein the plunger includes a recess opening toward the pressing operation member, and the pressing operation member includes a protrusion protruding toward the recess, and opposite ends of the coil spring are positioned by the recess of the plunger and the protrusion of the pressing operation member.

According to the drug solution injection controller structured following the present preferred embodiment, the coil spring can be held in an arranged state extending between the plunger and the pressing operation member by a simple structure in which the recess of the plunger and the protrusion of the pressing operation member are provided.

Further, for example, it would also be acceptable that the protrusion of the pressing operation member moved to the pushed position by the pressing operation is inserted into the recess of the plunger. With this configuration, in the compact drug solution injection controller, it is possible to sufficiently secure the stroke of the pressing operation of the pressing operation member.

A fourth preferred embodiment of the present invention provides the drug solution injection controller according to any one of the first to third preferred embodiments, wherein the outflow side flow path is configured to be closed by being pressed by the rapid administration valve in a direction orthogonal to the direction of movement of the pressing operation member.

According to the drug solution injection controller structured following the present preferred embodiment, by arranging the rapid administration valve on the lateral side of the outflow side flow path, the size of the housing can be reduced in the direction of movement of the pressing operation member.

A fifth preferred embodiment of the present invention provides the drug solution injection controller according to any one of the first to fourth preferred embodiments, wherein the priming flow path is configured to be closed by being pressed by the closing valve in a direction orthogonal to the direction of movement of the pressing operation member.

According to the drug solution injection controller structured following the present preferred embodiment, by arranging the closing valve on the lateral side of the priming flow path, the size of the housing can be reduced in the direction of movement of the pressing operation member.

A sixth preferred embodiment of the present invention provides the drug solution injection controller according to any one of the first to fifth preferred embodiments, wherein the priming flow path extends straightly from the sub-reservoir in the direction of movement of the pressing operation member.

According to the drug solution injection controller structured following the present preferred embodiment, the priming flow path whose flow path diameter is relatively large in order to complete the priming in a short time has a straight shape. This configuration obviates the process for bending the priming flow path and avoids increase in the number of components, thereby facilitating the production of the priming flow path.

Since the priming flow path extends in the direction of movement of the pressing operating member, the drug solution injection controller can also be downsized in the direction orthogonal to the direction of movement of the pressing operating member.

A seventh preferred embodiment of the present invention provides the drug solution injection controller according to any one of the first to sixth preferred embodiments, wherein the housing has an elongated shape, and an outer peripheral surface of the housing comprises a gripping surface to be gripped by a hand of a user, the pressing operating member is provided on one side in a longitudinal direction of the housing, and is movable back and forth in the longitudinal direction of the housing, and the pressing operation surface serves as a fingertip pressing surface that the user presses with a thumb on a distal end surface of the pressing operation member.

According to the drug solution injection controller structured following the present preferred embodiment, the user can apply a sufficient force to the pressing operation surface by pressing the pressing operation member with the thumb while gripping the gripping surface with one hand. Thus, rapid drug solution administration can be reliably performed by self-operation.

Moreover, the pressing operation surface of the pressing operation member, the sub-reservoir, and the flow path arrangement space of the inflow side flow path and the outflow side flow path of the drug solution with respect to the sub-reservoir are provided side by side in series in the longitudinal direction of the housing. Therefore, the housing can be made compact in the direction orthogonal to the longitudinal direction, thereby making the gripping surface thin.

An eighth preferred embodiment of the present invention provides he drug solution injection controller according to any one of the first to seventh preferred embodiments, wherein a cross-sectional shape of the housing in a direction orthogonal to the direction of movement of the pressing operation member is a flat hollow cross-sectional shape.

According to the drug solution injection controller structured following the present preferred embodiment, a large space in the housing can be secured in the major axis direction in the cross section of the housing. In the minor axis direction in the cross section of the housing, the housing can be downsized. Thus, for example, when carried in a pocket by the user, the drug solution injection controller is even less likely to be bulky. Besides, the housing having a flat cross-sectional shape is easy to grip.

A ninth preferred embodiment of the present invention provides the drug solution injection controller according to any one of the first to eighth preferred embodiments, wherein the closing valve has a plate-shaped valve body including an inclined contact side for closing the priming flow path so as to crush the priming flow path.

According to the drug solution injection controller structured following the present preferred embodiment, the contact portion of the closing valve with the priming flow path is the inclined contact side inclined with respect to the direction of approach of the closing valve to the priming flow path. Therefore, the repulsive force of the priming flow path acting in the direction of pushing back the closing valve is reduced in comparison with the case where the contact portion of the closing valve with the priming flow path is orthogonal to the direction of approach to the priming flow path. As a result, the closing valve can be switched to the closed state to close the priming flow path with a relatively small operating force.

A tenth preferred embodiment of the present invention provides the drug solution injection controller according to any one of the first to ninth preferred embodiments, further comprising a flow rate control part connecting the inflow side flow path and the outflow side flow path so as to continuously administer the drug solution without passing through the sub-reservoir.

According to the drug solution injection controller structured following the present preferred embodiment, the flow rate control par is provided in the drug solution injection controller. Thus, in the drug solution administration device including the drug solution injection controller, the external line from the main reservoir to the drug solution injection controller and the external line from the drug solution injection controller to the drug solution administration port on the patient side can both have a simple structure.

Moreover, by connecting the drug solution injection controller to the external line, the flow rate control part can be easily provided in the drug solution administration device.

An eleventh preferred embodiment of the present invention provides the drug solution injection controller according to the tenth preferred embodiment, wherein the flow rate control part is provided at a position farther from the pressing operation member than the limiting flow path, the priming flow path, and the closing valve are.

According to the drug solution injection controller structured following the present preferred embodiment, interference between the operating members such as the closing valve and the pressing operation member, and the flow rate control part constituting the line of the continuous administration of the drug solution is less likely to occur.

A twelfth preferred embodiment of the present invention provides the drug solution injection controller according to the tenth or eleventh preferred embodiment, wherein a cross-sectional shape of the housing in a direction orthogonal to the direction of movement of the pressing operation member is a flat hollow cross-sectional shape, a connecting flow path is provided so as to connect the inflow side flow path and the outflow side flow path while including the flow rate control part, and at least one of a direction of extension of the connecting flow path, a direction of movement of the rapid administration valve with respect to the outflow side flow path, and a direction of movement of the closing valve with respect to the priming flow path coincides with a major axis direction of a cross section of the housing.

According to the drug solution injection controller structured following the present preferred embodiment, the direction of extension of the connecting flow path, the direction of movement of the rapid administration valve, and the direction of movement of the closing valve coincide with the major axis direction of the cross section of the housing. Therefore, it is possible to secure a large space for arranging the connecting flow path and the space required for the stroke of the valve in the housing. In particular, if the direction of extension of the connecting flow path coincides with the major axis direction of the cross section of the housing, a large space for arranging the connecting flow path can be secured, thereby achieving a large degree of freedom in setting the length and the flow path cross-sectional area of the connecting flow path.

A thirteenth preferred embodiment of the present invention provides the drug solution injection controller according to any one of the tenth to twelfth preferred embodiments, wherein the inflow side flow path and the outflow side flow path include respective ports for connecting external flow paths, and a connecting flow path including the flow rate control part is provided so as to connect the ports.

According to the drug solution injection controller structured following the present preferred embodiment, the connecting flow path is arranged near the housing or is housed in the housing. Thus, the handleability of the connecting flow path can be improved as compared with the case where the connecting flow path is provided at the position remote from the housing in the external flow path.

A fourteenth preferred embodiment of the present invention provides the drug solution injection controller according to the thirteenth preferred embodiment, wherein the connecting flow path is arranged in the housing so as to curve toward a circumferential direction of the housing.

According to the drug solution injection controller structured following the present preferred embodiment, the connecting flow path can be housed in the housing with a good space efficiency. Further, since the connecting flow path is curved and arranged, the connecting flow path is less likely to bend (kink).

A fifteenth preferred embodiment of the present invention provides the drug solution injection controller according to any one of the tenth to fourteenth preferred embodiments, further comprising a regulating part for regulating movement of the flow rate control part.

According to the drug solution injection controller structured following the present preferred embodiment, the movement of the flow rate control part is regulated to some extent by the regulating part. Thus, when the flow rate control part is attached to the housing or when an impact force acts on the flow rate control part, bending (kink) of the flow rate control part or the like will be prevented.

A sixteenth preferred embodiment of the present invention provides the drug solution injection controller according to any one of the tenth to fifteenth preferred embodiments, wherein the flow rate control part is provided in the housing in a housed state.

According to the drug solution injection controller structured following the present preferred embodiment, since the flow rate control part is housed in the housing, the flow rate control part can be easily handled, and the flow rate control part is protected by the housing. The flow rate control part may be constituted by, for example, a small-diameter tube. In that case, the tubular flow rate control part can be more compactly housed in the housing in a state of being curved or wound at an arbitrary length.

### EFFECT OF THE INVENTION

According to the present invention, the drug solution injection controller can quickly complete the priming of the sub-line while being compact.

### BRIEF DESCRIPTION OF THE DRAWINGS

FIG. 1 is a view showing a drug solution administration device provided with a drug solution injection controller as a first practical embodiment of the present invention.
FIG. 2 is a view schematically showing the structure of the drug solution administration device shown in FIG. 1.
FIG. 3 is a perspective view of a drug solution injection controller constituting the drug solution administration device shown in FIG. 1.
FIG. 4 is a plan view of the drug solution injection controller shown in FIG. 3.
FIG. 5 is a front view of the drug solution injection controller shown in FIG. 3.
FIG. 6 is a perspective view of the drug solution injection controller shown in FIG. 3 in a state where the upper half portion of the housing is removed.
FIG. 7 is a perspective view of the drug solution injection controller shown in FIG. 3 in a state where the lower half portion of the housing is removed.
FIG. 8 is a cross-sectional view taken along line 8-8 of FIG. 5.
FIG. 9 is a cross-sectional view taken along line 9-9 of FIG. 4.
FIG. 10 is a cross-sectional view taken along line 10-10 of FIG. 4.
FIG. 11 is a cross-sectional view taken along line 11-11 of FIG. 4.
FIG. 12 is a view corresponding to the cross section taken along line 12-12 of FIG. 4.
FIG. 13 is a cross-sectional view taken along line 13-13 of FIG. 4.
FIG. 14 is a view corresponding to the cross section taken along line 14-14 of FIG. 4, which shows the state after a switch member is pushed.
FIG. 15 is a view corresponding to the cross section taken along line 15-15 of FIG. 4, which shows the state immediately after a push button is pushed.
FIG. 16 is a view corresponding to the cross section taken along line 16-16 of FIG. 4, which shows the state immediately after the push button is pushed.
FIG. 17 is a view corresponding to the cross section taken along line 17-17 of FIG. 4, which shows the state immediately after the push button is pushed.
FIG. 18 is a view corresponding to the cross section taken along line 18-18 of FIG. 5, which shows the state where rapid administration of a drug solution is completed.
FIG. 19 is a perspective view showing a drug solution injection controller as a second practical embodiment of the present invention, which shows the state where the lower half part of the housing is removed.
FIG. 20 is a cross-sectional view of the drug solution injection controller shown in FIG. 19, which shows the state where a switch member is pushed and a push button is not pushed.
FIG. 21 is another cross-sectional view of the drug solution injection controller shown in FIG. 20.
FIG. 22 is a cross-sectional view of the drug solution injection controller shown in FIG. 19, which shows the state where the switch member is pushed and the push button is pushed.
FIG. 23 is another cross-sectional view of the drug solution injection controller shown in FIG. 22.

### EMBODIMENTS FOR CARRYING OUT THE INVENTION

Hereinafter, practical embodiments of the present invention will be described with reference to the drawings.

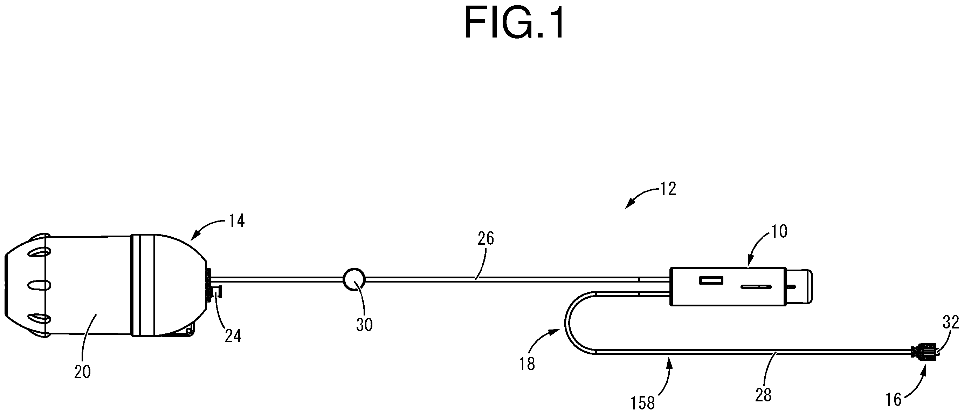

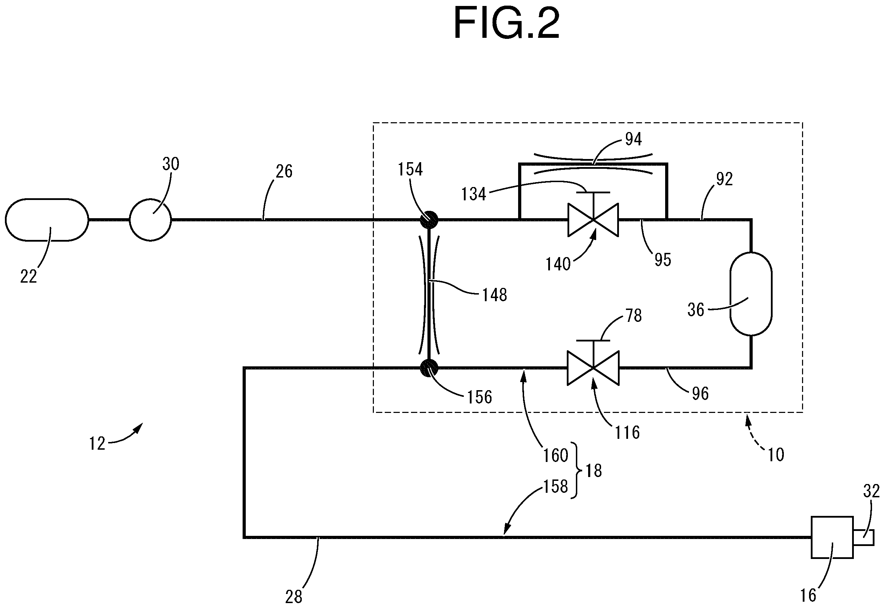

FIGS. 1 and 2 depict a drug solution administration device 12 provided with a drug solution injection controller 10 according to a first practical embodiment of the present invention. The drug solution administration device 12 has a structure in which a drug solution container 14 and a drug solution administration port 16 are connected by a drug solution administration line 18.

The drug solution container 14 has a structure in which a main reservoir 22 is housed in a rigid housing 20. The main reservoir 22 is a balloon formed of an elastic body such as rubber and resin elastomer. By a drug solution being injected from a syringe or the like (not shown) connected to a drug solution injection part 24, the main reservoir 22 is able to store the drug solution in a pressurized state. The drug solution stored in the main reservoir 22 will be pushed out from the main reservoir 22 based on the elasticity of the main reservoir 22.

However, the specific structure of the drug solution container is not particularly limited. For example, it would be acceptable that the drug solution container includes a rigid container body, a piston that is movable straightly in the container body, and an urging means such as a coil spring that urges the piston in the direction of movement, and a main reservoir for storing the drug solution is constituted between the container main body and the piston. The piston may be moved by the urging force of the urging means, so that the volume of the main reservoir gradually decreases and the drug solution is pushed out from the main reservoir.

Besides, the drug solution administration line 18 is connected to the main reservoir 22 of the drug solution container 14, and the drug solution stored in the main reservoir 22 of the drug solution container 14 will be delivered to the drug solution administration line 18. The drug solution administration line 18 includes an upstream side external line 26 and a downstream side external line 28. A filter 30 for filtering foreign matters in the drug solution is provided in the middle portion of the upstream side external line 26. The filter 30 may include both a filter for removing foreign matters and a ventilation filter for discharging air to the outside of the drug solution flow path.

Furthermore, the downstream side end of the downstream side external line 28 is connected to the drug solution administration port 16. The drug solution administration port 16 has a connector structure that can be connected to an indwelling needle or the like (not shown) that is percutaneously indwelled in a patient's blood vessel or the like. Moreover, in the present practical embodiment, a cap 32 provided with a breathable filter is attached to the drug solution administration port 16, so that the drug solution administration port 16 prevents the drug solution from flowing out to the outside while allowing the air to be discharged to the outside.

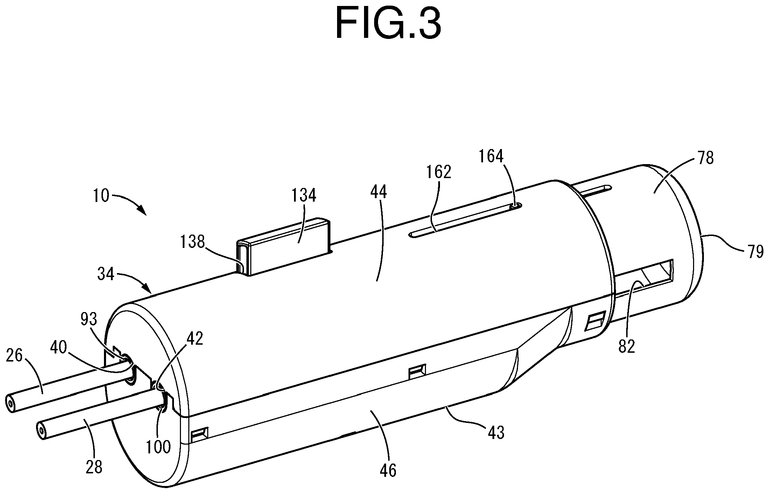

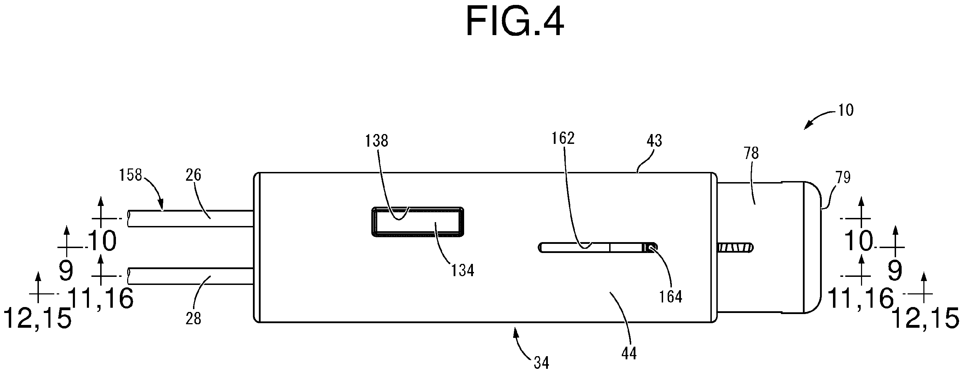

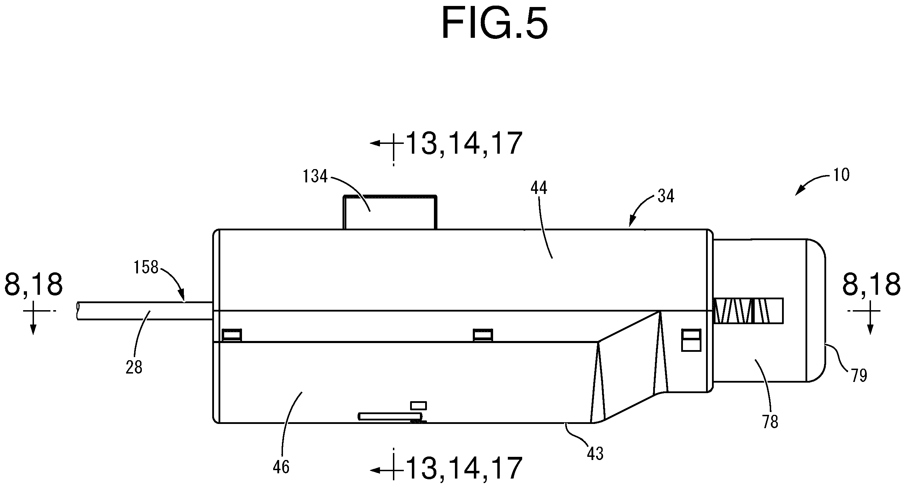

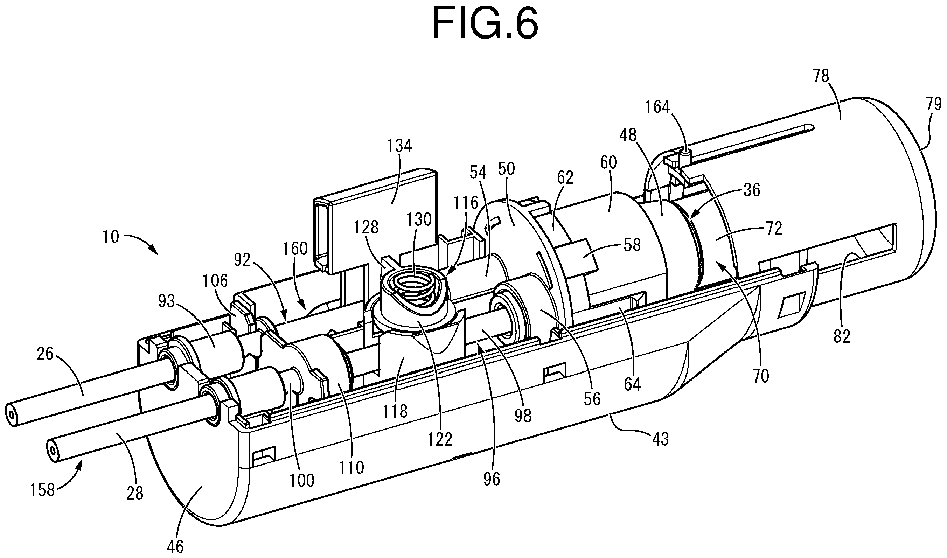

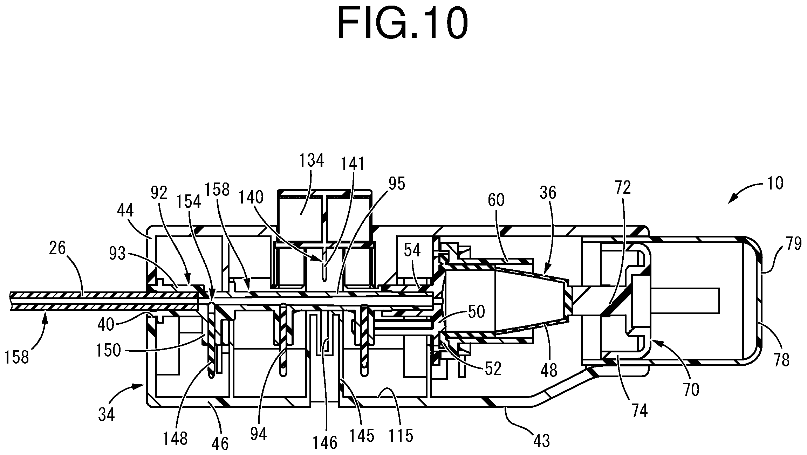

Further, the upstream side external line 26 and the downstream side external line 28 are connected to the drug solution injection controller 10 as shown in FIGS. 3 to 13. The drug solution injection controller 10 is for performing rapid administration of a drug solution by self-operation, and has a structure in which a sub-reservoir 36 is incorporated in a housing 34 in a housed state as shown in FIGS. 6 to 12. In the following description of the drug solution injection controller 10, as a general rule, the vertical direction refers to the vertical direction in FIG. 5, the front-back direction refers to the left-right direction which coincides with the axial direction in FIG. 4, and the left-right direction refers to the vertical direction in FIG. 4.

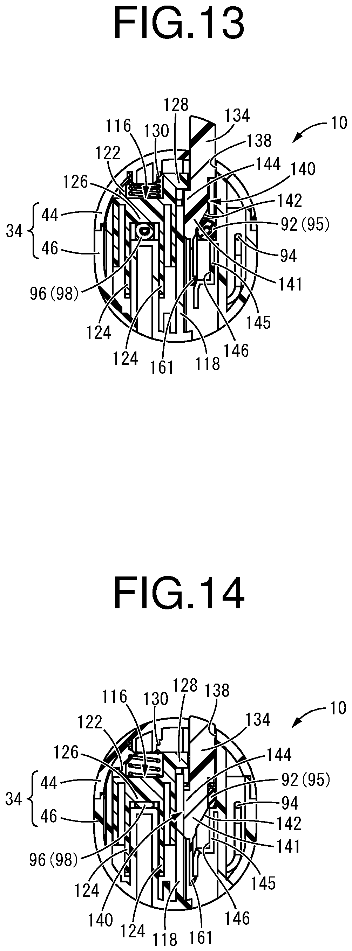

More specifically, the housing 34 is made of a rigid synthetic resin or the like, and has a hollow shape elongated in the front-back direction overall, and more specifically, a generally bottomed tubular shape. As shown in FIGS. 13 and 14, the housing 34 has a flat hollow cross-sectional shape over a wide range excluding the back end portion, and in the present practical embodiment, the housing 34 has a hollow, substantially elliptical cross section. With this configuration, in the housing 34, the major axis direction, which is the longitudinal direction in the cross section, coincides with the vertical direction, and the minor axis direction coincides with the left-right direction. However, the flat cross-sectional shape referred to here is not limited to an elliptical cross section, and may be, for example, a flat polygonal cross section or the like. That is, the expression that the housing 34 has the flat cross-sectional shape means that the housing 34 has the cross-sectional shape having directionality so as to be crushed in one direction. Typically, since the inscribed circle or the circumscribed circle in the cross section has an oval, an elliptical, or a shape of a similar kind, the width dimensions in the two orthogonal directions in the flat cross section are different from each other, and the direction whose width dimension is larger (the longitudinal direction) refers to the major axis direction. Specifically, for example, in the case of a rectangular cross section in which the lengths of two sets of opposite sides are different from each other, the long-side direction of the rectangle refers to the longitudinal direction or the major axis direction.

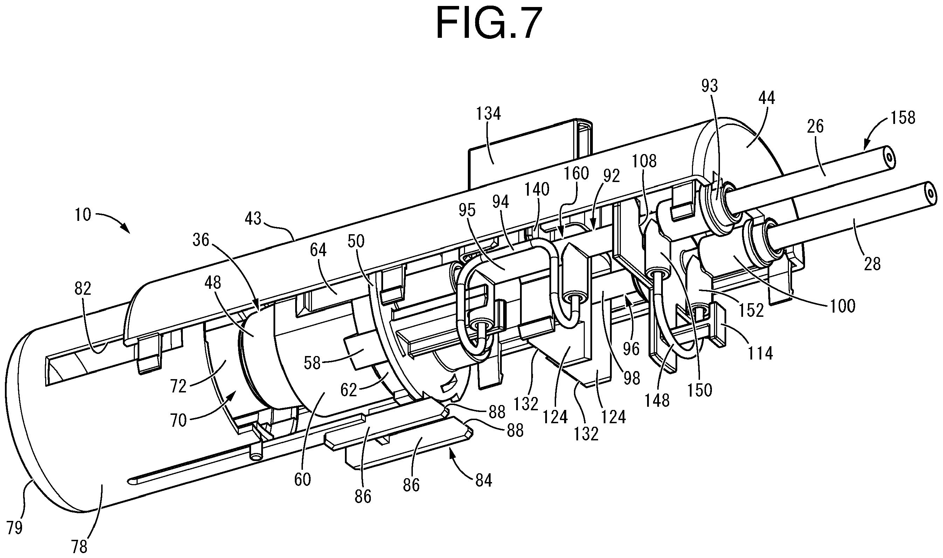

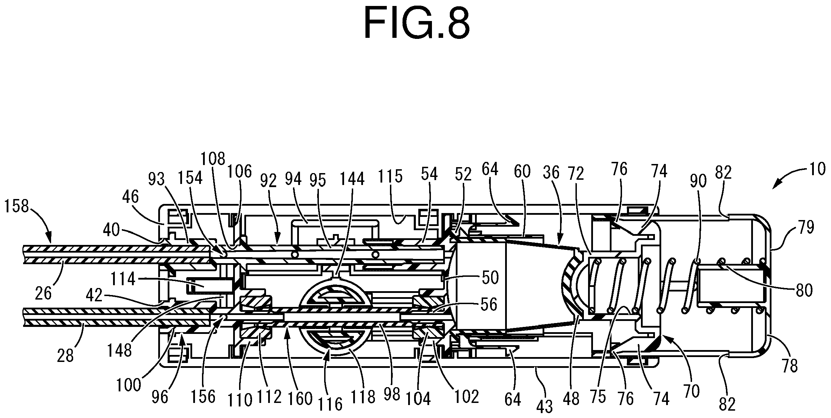

Two insertion holes 40, 42 are formed so as to pass through the wall portion on the front side (the left side in FIG. 8) of the housing 34 in the longitudinal direction. Preferably, the housing 34 has such an outer diameter dimension that the user can grip the housing 34 with one hand, and the outer peripheral surface is provided with a gripping surface 43 to be gripped by the user's hand. The housing 34 of the present practical embodiment is constituted by combining an upper half portion 44 and a lower half portion 46 in the vertical direction. In order to make the internal structure of the drug solution injection controller 10 easier to see, the upper half portion 44 of the housing 34 is omitted in FIG. 6, and the lower half portion 46 of the housing 34 is omitted in FIG. 7.

Besides, the sub-reservoir 36 is housed in the housing 34. As shown in FIGS. 8 to 11, the sub-reservoir 36 is constituted by covering the opening of a cup-shaped diaphragm part 48 with a base member 50.

The diaphragm part 48 is made of rubber, a resin elastomer, or the like and has flexibility. The diaphragm part 48 has a generally cup shape in a stationary state where no external force acts, and a flange-shaped clasped part 52 protruding to the outer periphery is integrally formed with the opening portion of diaphragm part 48 over the entire circumference.

The base member 50 is made of a rigid synthetic resin or the like and has a generally circular disk shape, and is penetrated by a tubular drug solution inflow part 54 and a tubular drug solution outflow part 56. Moreover, a plurality of locking protrusions 58 protrude backward (to the right in FIG. 8) on the base member 50, and the base member 50 is connected to a guide member 60 by the locking protrusions 58.

The guide member 60 has a generally cylindrical shape, and is provided with a flange-shaped locking part 62 that protrudes toward the outer periphery at the front end part thereof. By the locking protrusions 58 of the base member 50 being hooked on the locking part 62, the base member 50 and the guide member 60 are connected in the axial direction. Besides, the locking part 62 of the guide member 60 is provided with engaging hooks 64 at two locations in the circumferential direction. Each engaging hook 64 integrally includes a plate-shaped extending part extending backward from the locking part 62 and a claw part protruding from the distal end of the extending part toward the outer periphery.

The diaphragm part 48 is arranged on the radially inner side of the guide member 60. The diaphragm part 48 is arranged on the inner circumference of the guide member 60, and the clasped part 52 is sandwiched and supported between the base member 50 and the guide member 60 in the axial direction (the front-back direction), whereby the diaphragm part 48 is attached to base member 50 and the guide member 60. In such an attached state, the opening of the diaphragm part 48 is liquid-tightly closed by the base member 50 to form the sub-reservoir 36. Furthermore, the internal space of the sub-reservoir 36 communicates with the lumens of the drug solution inflow part 54 and the drug solution outflow part 56 penetrating the base member 50.

Then, the base member 50 and the guide member 60 are supported by the housing 34, so that the sub-reservoir 36 is positioned with respect to the housing 34, and the diaphragm part 48 is allowed to deform so as to move its bottom part in the front-back direction with respect to the housing 34. The sub-reservoir 36 is arranged behind the center in the front-back direction of the housing 34.

A plunger 70 is overlapped on the bottom part of the diaphragm part 48. The plunger 70 has a structure in which a plunger main body 72 having a generally round tubular shape with a bottom and a tubular guide tube portion 74 provided so as to surround the outer circumference of the plunger main body 72 are integrally connected at the back end part. The plunger main body 72 includes a recess 75 opening backward. The recess 75 extends straightly with a circular cross section corresponding to the outer shape of a coil spring 90 described later. The guide tube portion 74 is partially thinned at two locations on the circumference and expanded to the outer periphery, and hook releasing parts 76, 76 are formed by the thinned portions. The hook releasing parts 76, 76 are provided at positions corresponding to the engaging hooks 64, 64 of the guide member 60 in the circumferential direction.

Further, the plunger 70 is inserted in a push button 78 serving as a pressing operation member. The push button 78 has a generally round tubular shape with a bottom that opens toward the front. The opening portion of the push button 78 is inserted from the back side in the axial direction with respect to the back end portion of the housing 34 and attached so as to be movable back and forth in the longitudinal direction (the front-back direction) of the housing 34. The bottom side of the push button 78 projects axially backward from the back end opening of the housing 34. The back surface of the push button 78 exposed from the housing 34 comprises a pressing operation surface 79 serving as a fingertip pressing surface, and the user can perform a pressing operation with a thumb while grasping the gripping surface 43 of the housing 34. Further, a spring support part 80 serving as a protrusion is integrally formed on the radially inner portion of the bottom part of the push button 78. The spring support part 80 has a small-diameter tubular shape having an outer shape corresponding to the inner shape of the coil spring 90 described later, and projects forward from the central portion of the bottom wall part of the push button 78. Besides, the outer diameter dimension of the spring support part 80 is smaller than the inner diameter dimension of the recess 75. Furthermore, hook engaging holes 82 extending in the axial direction are formed at two locations in the circumferential direction on the peripheral wall part of the push button 78. The distal end of the hook releasing part 76 of the plunger 70 is inserted into the hook engaging hole 82 and locked to the radially inner surface of the hook engaging hole 82.

Moreover, as shown in FIG. 7, a main drive piece 84 is integrally formed with the push button 78. The main drive piece 84 projects forward from the opening end of the push button 78 and extends to the outer periphery of the guide member 60. Furthermore, the projecting distal end portion of the main drive piece 84 comprises two sliding contact parts 86, 86 arranged in parallel, and the projecting distal end surfaces of the sliding contact parts 86, 86 comprise inclined surfaces 88 that incline in the vertical direction toward the distal end side. Since the inclined surface 88 of the present practical embodiment is provided on the upper portion of the sliding contact part 86 and has a shape that inclines downward toward the projecting distal end, the sliding contact part 86 has a tapered shape. The two sliding contact parts 86, 86 may have the same shape or may have different shapes from each other.

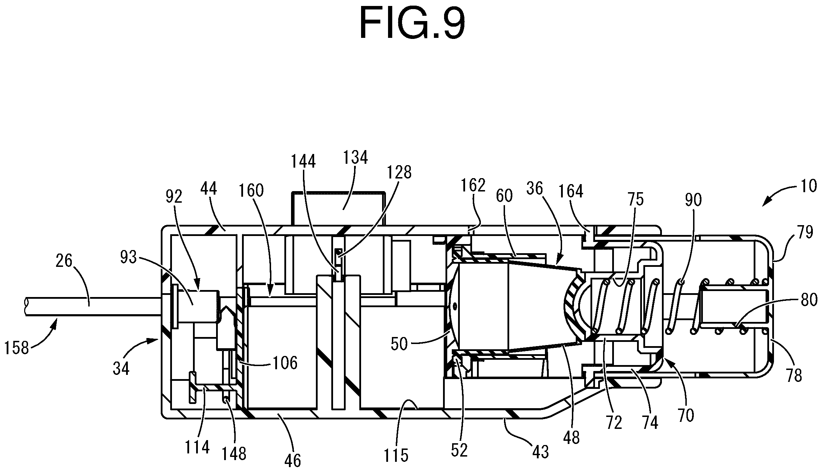

Besides, as shown in FIGS. 8 and 9, a coil spring 90 is arranged between the plunger 70 and the push button 78 in the axial direction. The coil spring 90 is arranged such that its front end is inserted into the radial inside of the recess 75 of the plunger main body 72, while its back end is placed externally about a spring support part 80 protruding from the bottom wall part of the push button 78, so as to be positioned in the axis-perpendicular direction. With this configuration, the coil spring 90 is stably held in a predetermined arrangement state extending between the plunger main body 72 and the push button 78 in the front-back direction.

On the other hand, to the base member 50, an inflow side tube 92 serving as an upstream side connector is connected. The inflow side tube 92 is a soft member made of rubber, a resin elastomer, or the like. As shown in FIGS. 6 to 8 and 10, the inflow side tube 92 is provided with an inflow side port 93 which is a port for connecting an external flow path at the front end portion, and the inflow side port 93 is fitted into the insertion hole 40 of the housing 34. The upstream side external line 26 is fitted into the inflow side port 93 of the inflow side tube 92, while the back end part of the inflow side tube 92 is fitted into the drug solution inflow part 54 of the base member 50. With this configuration, the upstream side external line 26 and the sub-reservoir 36 are connected by the inflow side tube 92 in an open state.

Further, as shown in FIGS. 7 and 8, to the inflow side tube 92, an orifice tube 94 serving as a limiting flow path is connected. The orifice tube 94 is provided in parallel with the middle portion of the inflow side tube 92 in the housing 34, and constitutes a bypassing flow path that connects two locations that are remote from each other in the flow path direction of the inflow side tube 92 on the upstream side of the sub-reservoir 36. The inflow side tube 92 provides a priming flow path 95 extending in parallel with the orifice tube 94 between the portions connected to the orifice tube 94. The priming flow path 95 of the present practical embodiment is provided to the inflow side tube 92 extending forward from the sub-reservoir 36, and extends straightly in the front-back direction.

Besides, to the base member 50, an outflow side tube 96 serving as a downstream side connector is connected. The outflow side tube 96 extends in the front-back direction overall. As shown in FIGS. 6 to 8 and 11, the outflow side tube 96 includes a connection tube 98 connected to the drug solution outflow part 56 of the base member 50, and an outflow side port 100, which is a port for connecting an external flow path and is connected in series with the connection tube 98.

The connection tube 98 is a soft member made of rubber, a resin elastomer, or the like, extends straightly in the axial direction, and is externally fitted to the drug solution outflow part 56 of the base member 50. The base member 50 is provided with a tubular seal holding part 102 so as to surround the periphery of the drug solution outflow part 56. By an annular sealing member 104 being fitted between the connection tube 98 fitted externally onto the drug solution outflow part 56 and the seal holding part 102, the connection tube 98 is pressed against the drug solution outflow part 56, thereby improving sealing property.

The outflow side port 100 extends straightly in the axial direction and is fitted into the front end part of the connection tube 98, so as to be connected in series with the connection tube 98. Besides, the outflow side port 100 is provided with a plate-shaped connection piece 106 extending in the axis-perpendicular direction in the middle portion. The inflow side tube 92 provided in parallel with the outflow side tube 96 is fitted into a concave part 108 of the connection piece 106 and is positioned. Furthermore, the connection piece 106 is provided with a tubular seal holding part 110 so as to surround the periphery of the back end part of the outflow side port 100. By an annular sealing member 112 being fitted between the connection tube 98 fitted externally onto the outflow side port 100 and the seal holding part 110, the connection tube 98 is pressed against the outflow side port 100, thereby improving sealing property. Moreover, the connection piece 106 is integrally includes a latching protrusion 114 serving as a regulating part that projects forward between the inflow side tube 92 and the outflow side tube 96.

Regarding the outflow side tube 96 constituted by the connection tube 98 and the outflow side port 100, the front end part of the outflow side port 100 is inserted through the insertion hole 42 of the housing 34. The downstream side external line 28 is fitted into the front end part of the outflow side port 100. Further, the back end part of the connection tube 98 of the outflow side tube 96 is externally fitted onto the drug solution outflow part 56 of the base member 50.

That is, a flow path arrangement space 115 is provided in front of the sub-reservoir 36. In the present practical embodiment, since the sub-reservoir 36 is arranged behind the center in the front-back direction of the housing 34, the flow path arrangement space 115 is widely obtained. The inflow side tube 92, which is the inflow side flow path of the drug solution with respect to the sub-reservoir 36, and the outflow side tube 96, which is the outflow side flow path of the drug solution with respect to the sub-reservoir 36, are arranged in the flow path arrangement space 115. Further, the orifice tube 94 and the priming flow path 95 that bypasses the orifice tube 94 are also arranged in the flow path arrangement space 115. The inflow side tube 92 including the priming flow path 95 and the outflow side tube 96 are provided side by side in the left-right direction of the housing 34, and are arranged so as to generally follow the direction of movement of the push button 78.

The flow path arrangement space 115, the sub-reservoir 36, and the pressing operation surface 79 of the push button 78 are provided side by side in series in the longitudinal direction of the housing 34 (the left-right direction in FIG. 8), which is the direction of moving back and forth of the push button 78. The inflow side tube 92 and the outflow side tube 96 extend in the generally axial direction. Further, the pressing operation surface 79 of the push button 78 extends in the generally axis-perpendicular direction.

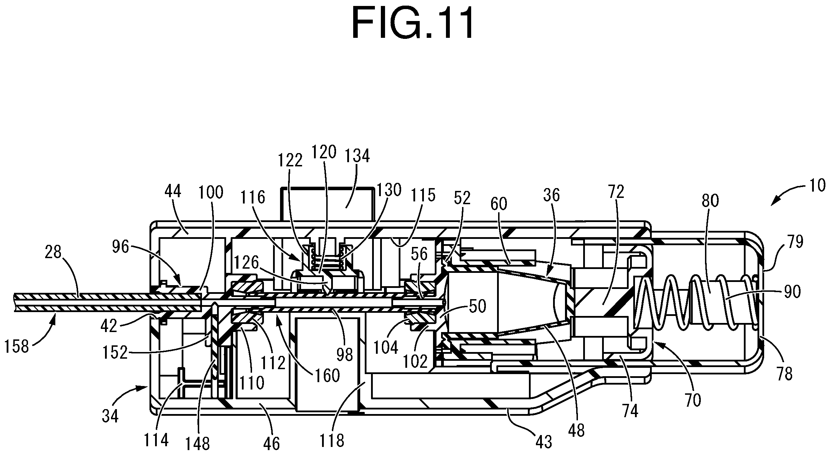

Besides, as shown in FIGS. 6 and 11, a rapid administration valve 116 is arranged above the middle portion of the outflow side tube 96. The rapid administration valve 116 is inserted into a tubular valve guide part 118 provided in the lower half portion 46 of the housing 34, and is attached in a condition such that relative displacement in the vertical direction with respect to the housing 34 is allowed. More specifically, as shown in FIGS. 11 to 13, in the rapid administration valve 116, a tubular spring guide part 122 projecting to the upper side is integrally formed with a valve main body 120 having a generally circular disk shape. Further, a pair of follower pieces 124, 124 to be inserted into the valve guide part 118 are integrally formed so as to project to the lower side from the valve main body 120.

The valve main body 120 is provided with a flat plate-shaped clamp protrusion 126 projecting downward in the front-back central portion so as to spread generally orthogonally to the front-back direction, and the distal end portion of the clamp protrusion 126 is gradually thinned toward the tip. Moreover, the valve main body 120 is provided with a switch engaging protrusion 128 projecting to the outer periphery in a part in the circumferential direction (see FIG. 6).

A coil spring 130 serving as a valve urging means is inserted into the spring guide part 122, and the coil spring 130 inserted into the spring guide part 122 is compressed between the valve main body 120 and the upper half portion 44 of the housing 34 in the vertical direction. With this configuration, a downward urging force by the coil spring 130 acts on the rapid administration valve 116.

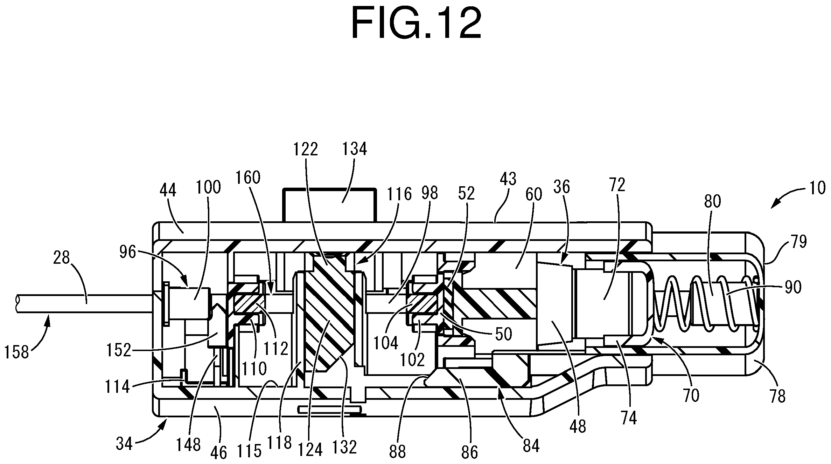

As shown in FIGS. 7 and 12, the follower piece 124 has a plate shape extending in the vertical direction, and its lower surface comprises an inclined surface 132 that inclines upward toward the back. The pair of follower pieces 124 are arranged at a predetermined distance in the left-right direction, and a clamp protrusion 126 is provided between the proximal ends of the pair of follower pieces 124, 124. A part of the outflow side tube 96 extends back and forth between the pair of follower pieces 124, 124, and the valve main body 120 provided with the clamp protrusion 126 is located above the outflow side tube 96.

Additionally, as shown in FIG. 9, the valve main body 120 of the rapid administration valve 116 is held above the outflow side tube 96 against the urging force of the coil spring 130 by the switch engaging protrusion 128 being vertically engaged with an engagement receiver 144 (described later) which is integrally provided on the switch member 134. The rapid administration valve 116 is arranged in the flow path arrangement space 115 together with the outflow side tube 96. The rapid administration valve 116 is arranged on the lateral side (upper side) of the outflow side tube 96, and moves in the vertical direction that is orthogonal to the direction of moving back and forth of the push button 78 as described later. Therefore, the drug solution injection controller 10 can be downsized in the front-back direction and in the left-right direction. Regarding the housing portion of the rapid administration valve 116 in the housing 34, the cross section orthogonal to the front-back direction has a flat shape in which the width dimension in the vertical direction is larger than the width dimension in the left-right direction. This makes it also possible to obtain a sufficient moving stroke of the rapid administration valve 116 that moves in the vertical direction.

As shown in FIGS. 3 to 5, the switch member 134 has a generally rectangular box shape overall. The switch member 134 is inserted through the switch insertion hole 138 provided in the upper half portion 44 of the housing 34, and is positioned in the front-back direction and in the left-right direction with respect to the housing 34, while being displaceable relative to the housing 34 in the vertical direction.

Moreover, the switch member 134 includes a closing valve 140. The closing valve 140 is arranged in the flow path arrangement space 115 of the housing 34. The closing valve 140 includes a plate-shaped valve body 141 that projects downward. The valve body 141 extends generally orthogonally to the front-back direction, which is the length direction of the priming flow path 95. The valve body 141 is arranged between the opposite ends of the orifice tube 94 in the front-back direction. In other words, the orifice tube 94 is provided so as to connect the upstream side and the downstream side of the portion where the valve body 141 is arranged in a sub-line 160 described later.

Besides, the valve body 141 is arranged above the inflow side tube 92 in the initial state before the switch member 134 is pushed in, and the lumen of the inflow side tube 92 is in an open state without being crushed by the valve body 141 of the closing valve 140. The closing valve 140 is arranged on the lateral side (upper side) of the inflow side tube 92, and moves downward in the vertical direction that is orthogonal to the direction of moving back and forth of the push button 78 as described later. Therefore, the drug solution injection controller 10 can be downsized in the front-back direction and in the left-right direction. Regarding the housing portion of the closing valve 140 in the housing 34, the cross section orthogonal to the front-back direction has a flat shape in which the width dimension in the vertical direction is larger than the width dimension in the left-right direction. This makes it also possible to obtain a sufficient moving stroke of the closing valve 140 that moves in the vertical direction.

Further, an inclined contact side 142 forming the lower surface of the valve body 141 is arranged in opposition to the upper side of the inflow side tube 92, and the inclined contact side 142 comprises an inclined surface inclining downward from the right side to the left side in FIG. 13. It is desirable that the inclined contact side 142 has an inclined angle set within a range of 10° to 80° with respect to the vertical direction in FIG. 13, which is the direction of movement of the valve body 141. Moreover, the closing valve 140 is provided with an engagement receiver 144 that projects in the left-right direction and engages with the switch engaging protrusion 128 of the rapid administration valve 116 in the vertical direction.

The lower half portion 46 of the housing 34 is provided with a tube support part 145 that projects upward at a portion that faces the closing valve 140 in the vertical direction. The tube support part 145 abuts and supports a part of the inflow side tube 92 from below. Further, in the tube support part 145, a recess 146 that opens upward is formed in the middle portion in the front-back direction corresponding to the closing valve 140.

Additionally, as shown in FIG. 7, the inflow side port 93 of the inflow side tube 92 and the outflow side port 100 of the outflow side tube 96 are connected by a flow rate control tube 148 serving as a flow rate control part. The flow rate control tube 148 is a flexible tube made of rubber, a resin elastomer, or the like, and has a smaller diameter than those of the inflow side tube 92 and the outflow side tube 96. The front end part of the flow rate control tube 148 is inserted into a tubular connecting part 150 provided to the inflow side port 93 and communicates with the inflow side port 93, while the back end part of the flow rate control tube 148 is inserted into the tubular connecting part 152 provided to the outflow side port 100 and communicates with the outflow side port 100. With this configuration, the connecting flow path connecting the inflow side port 93 and the outflow side port 100 is constituted by the flow rate control tube 148. In the present practical embodiment, the connecting flow path constituted by the flow rate control tube 148 has a generally constant flow path diameter throughout, and the entire connecting flow path comprises the flow rate control part. However, by the connecting flow path being partially reduced in diameter, the flow rate control part may be partially provided with respect to the connecting flow path.

The connecting parts 150, 152 are provided in front of the connection piece 106 of the outflow side tube 96, and the opposite ends of the flow rate control tube 148 are respectively connected to the inflow side port 93 constituting the front part of the inflow side tube 92 and the outflow side port 100 constituting the front part of the outflow side tube 96. Further, the connecting parts 150, 152 are branched downward from the inflow side port 93 and the outflow side port 100, and extend downward, which is the major axis direction in the cross section of the housing 34. With this configuration, the connecting parts 150, 152 and the flow rate control tube 148 are arranged so as to extend in the major axis direction of the cross section that can secure a wide space in the housing 34, and are housed in the housing 34 with a good space efficiency.

Besides, the flow rate control tube 148 is provided at a position farther from the push button 78 than the orifice tube 94, the priming flow path 95, and the closing valve 140 are. In other words, the flow rate control tube 148 is arranged on the opposite side and away from the push button 78 with respect to the orifice tube 94, the priming flow path 95, and the closing valve 140 in the front-back direction of the housing 34. Accordingly, the flow rate control tube 148 is arranged at a position away from the operating members such as the closing valve 140 and the push button 78 that move with respect to the housing 34, so that the flow rate control tube 148 and the operating members are less likely to interfere with each other. Therefore, it is possible to prevent the flexible and small-diameter flow rate control tube 148 from being damaged by contact with the operating members.

In the present practical embodiment, the upstream side end of the flow rate control tube 148 is inserted up to the proximal end portion of the connecting part 150, and is connected to a branch point 154 of the inflow side port 93 and the connecting part 150. Moreover, the downstream side end of the flow rate control tube 148 is inserted up to the proximal end portion of the connecting part 152, and is connected to the confluence point 156 of the outflow side port 100 and the connecting part 152. When the flow rate control tube 148 is connected to the branch point 154 and the confluence point 156, it is desirable that the opposite end openings of the flow rate control tube 148 are provided so as to directly communicate with the branch point 154 and the confluence point 156. However, the opposite end openings of the flow rate control tube 148 may be arranged at a position close to the branch point 154 and the confluence point 156, so as to indirectly communicate with the branch point 154 and the confluence point 156 via the connecting parts 150, 152. When the opposite end openings of the flow rate control tube 148 are connected close to the branch point 154 and the confluence point 156, it is desirable that the opposite ends of the flow rate control tube 148 are inserted so as to reach the positions on the proximal sides of the connecting parts 150, 152 where the air remaining in the lumens of the connecting parts 150, 152 after priming, which will be described later, does not matter. More specifically, the opposite ends of the flow rate control tube 148 are arranged close to the branch point 154 and the confluence point 156 such that, in the lumens of the connecting parts 150, 152, the volume of the region from the proximal ends of the connecting parts 150, 152 to the ends of the flow rate control tube 148 is 3 ml or less, preferably 1 ml or less, for example.

Furthermore, the flow rate control tube 148 has a generally U-shape overall, extends downward from the connecting parts 150, 152, and is arched and extend so as to turn below the latching protrusion 114 protruding from the connection piece 106. With this configuration, the flow rate control tube 148 is incorporated in the housing 34 in a housed state in its entirety, and at least the middle portion of the flow rate control tube 148 curves toward the circumferential direction of the housing 34. By arranging the flow rate control tube 148 so as to curve toward the circumferential direction of the housing 34 in this way, the flow rate control tube 148 can be efficiently housed in the housing 34, and the flow rate control tube 148 is less likely to bend and cause a problem. The length of the flow rate control tube 148 is set according to the required flow rate per unit time of a main line 158 (described later). When the length of the flow rate control tube 148 is long, the flow rate control tube 148 can also be held by being wound around the latching protrusion 114.

The drug solution injection controller 10 having such a structure constitutes the drug solution administration device 12 by the upstream side external line 26 being connected to the inflow side port 93 while the downstream side external line 28 being connect to the outflow side port 100.

Further, in the drug solution injection controller 10, the inflow side port 93 and the outflow side port 100 are communicated with each other by the flow rate control tube 148, so that the main line 158 of the drug solution administration device 12 connecting the main reservoir 22 and the drug solution administration port 16 is constituted. That is, the main line 158 of the drug solution administration device 12 is constituted by the upstream side external line 26 connected to the main reservoir 22, the inflow side port 93 connected to the upstream side external line 26, the flow rate control tube 148 connected to the inflow side port 93, the outflow side port 100 to which the flow rate control tube 148 is connected, and the downstream side external line 28 connected to the outflow side port 100. The main line 158 is constituted without including the sub-reservoir 36 in the path by the inflow side port 93 and the outflow side port 100 being communicated with each other by the flow rate control tube 148. Therefore, the drug solution stored in the main reservoir 22 can be continuously administered from the drug solution administration port 16 through the main line 158 without passing through the sub-reservoir 36. The main line 158 of the present practical embodiment is always in a communicating state, but for example, the main line 158 can be temporarily closed by appropriately clamping the external lines 26, 28.

Meanwhile, a sub-line 160 of the drug solution administration device 12 is constituted by the portion downstream of the connecting part 150 in the inflow side tube 92, the orifice tube 94 connected to the inflow side tube 92, and the portion upstream of the connecting part 152 in the outflow side tube 96. This sub-line 160 is provided so as to branch from the main line 158 at the branch point 154 of the inflow side tube 92 and the connecting part 150, and to join the main line 158 at the confluence point 156 of the outflow side tube 96 and the connecting part 152 via the sub-reservoir 36. In the present practical embodiment, the entire sub-line 160 is incorporated in the housing 34 in a housed state, and the branch point 154 and the confluence point 156 of the main line 158 and the sub-line 160 are incorporated in the housing 34 in a housed state.

The drug solution administration line 18 connecting the main reservoir 22 and the drug solution administration port 16 includes the main line 158 and the sub-line 160. Further, the upstream side external line 26 is provided by connecting the main reservoir 22 and the drug solution injection controller 10, and the downstream side external line 28 is provided by connecting the drug solution injection controller 10 and the drug solution administration port 16.

The drug solution administration device 12 provided with the drug solution injection controller 10 having such a structure is subjected to priming in which the main line 158 and the sub-line 160 are filled with the drug solution (priming liquid) before use. That is, in the initial state of the drug solution injection controller 10 in which the switch member 134 and the push button 78 are not pushed, the drug solution is sent from the main reservoir 22 to the drug solution administration line 18, so that the upstream side external line 26, the inflow side tube 92, the sub-reservoir 36, the outflow side tube 96, and the downstream side external line 28 are filled with the drug solution, and the air pushed to the downstream side by the drug solution is discharged to the outside through the cap 32 of the drug solution administration port 16. By so doing, in the present practical embodiment, the air is also discharged to the outside by the filter 30 provided to the upstream side external line 26. Accordingly, the priming of the main line 158 and the sub-line 160 is completed in the entirety except for the orifice tube 94 and the flow rate control tube 148, which have a small diameter and do not allow the drug solution to flow easily.

At the time of priming, the closing valve 140 is in the open state and the priming flow path 95 of the inflow side tube 92 is in the communicating state. Thus, the drug solution flows through the priming flow path 95, so that the priming of the main line 158 and the sub-line 160 is completed promptly.

Regarding the orifice tube 94 and the flow rate control tube 148, the volume of the lumen is extremely small, and the amount of air remaining inside is so small that it substantially does not matter. Thus, the discharge of air by priming may be obviated. In the present practical embodiment, the upstream side end of the flow rate control tube 148 is inserted up to the proximal end portion of the connecting part 150 and connected to the branch point 154 of the main line 158 and the sub-line 160, while the downstream side end of the flow rate control tube 148 is inserted up to the proximal end portion of the connecting part 152 and connected to the confluence point 156 of the main line 158 and the sub-line 160. This prevents air from remaining between the branch point 154 and the upstream side end of the flow rate control tube 148 and between the downstream side end of the flow rate control tube 148 and the confluence point 156, and the amount of air remaining in the drug solution administration line 18 after completion of priming is so small that it does not matter.

After the priming is completed, by pushing the switch member 134 downward, as shown in FIG. 14, the valve body 141 of the closing valve 140 integrated with the switch member 134 moves downward, and the inclined contact side 142 of the valve body 141 is pressed against the priming flow path 95 of the inflow side tube 92 in the direction orthogonal to the flow path of the priming flow path 95. By so doing, the inflow side tube 92 is crushed at the middle portion (the priming flow path 95) against which the valve body 141 of the closing valve 140 is pressed, and the inflow side tube 92 is closed at the priming flow path 95 pressed by the closing valve 140. Furthermore, regarding the inflow side tube 92, the upstream side and the downstream side of the portion of the priming flow path 95 closed by the closing valve 140 communicate with each other through the orifice tube 94. Therefore, in the state where the priming flow path 95 of the inflow side tube 92 is closed by the closing valve 140, the sub-line 160 is kept open from the branch point 154 to the sub-reservoir 36 by the orifice tube 94, and the flow rate per unit time of the drug solution is limited by the orifice tube 94.

In the closing valve 140 of the present practical embodiment, the portion that comes into contact with the inflow side tube 92 comprises the inclined contact side 142 having an inclined shape. Thus, when the closing valve 140 is pressed against the inflow side tube 92 and the inflow side tube 92 is crushed, the repulsive force based on the elasticity of the inflow side tube 92 acts in a dispersed manner not only in the vertical direction but also in the left-right direction. As a result, the repulsive force in the vertical direction becomes smaller than when the repulsive force acts only in the vertical direction, and the downward force to be exerted on the switch member 134 in order to push down the closing valve 140 can be reduced.

Moreover, in the present practical embodiment, in the open state of the closing valve 140, the inclined contact side 142 is in contact with the outer peripheral surface of the inflow side tube 92, and the lower end of the inclined contact side 142 is located below the contact area with the inflow side tube 92. Therefore, the effect exhibited by the closing valve 140 being pressed against the inflow side tube 92 at the inclined contact side 142 can be obtained more stably.

Furthermore, since the inflow side tube 92 after being crushed by the closing valve 140 is compressed in the left-right direction between the side surface of the closing valve 140 and the housing 34, the repulsive force based on the elasticity of the inflow side tube 92 is less likely to act upward on the closing valve 140. Therefore, the closing valve 140 is stably held in the closed state, and the closed state of the inflow side tube 92 and hence the sub-line 160 is stably maintained.

Additionally, when the switch member 134 moves downward, the engagement receiver 144 of the switch member 134 and the switch engaging protrusion 128 of the rapid administration valve 116 are disengaged, thereby allowing the rapid administration valve 116 to move downward. By so doing, the rapid administration valve 116 moves downward due to the urging force of the coil spring 130, and the clamp protrusion 126 of the rapid administration valve 116 is pressed against the connection tube 98 of the outflow side tube 96 in the direction orthogonal to the flow path of the outflow side tube 96. As a result, the outflow side tube 96 is partially crushed by the rapid administration valve 116, and the lumen of the outflow side tube 96 pressed by the rapid administration valve 116 is closed off, so that outflow of the drug solution from the sub-reservoir 36 is prevented by closing the outflow side tube 96.

As shown in FIG. 13, the switch member 134 pushed downward is configured to be held in the pushed state and prevented from returning upwards by the locking piece 161 projecting downward being locked to the tube support part 145 of the housing 34.

On the other hand, in a state where the switch member 134 is pushed down, the main line 158 is maintained in a communicating state. Then, the cap 32 is removed from the drug solution administration port 16 connected to the main line 158, and the drug solution administration port 16 is connected to an indwelling needle or the like indwelled in the blood vessel of the patient. By so doing, the drug solution sent from the main reservoir 22 to the main line 158 is sent to the drug solution administration port 16 without passing through the sub-line 160, and is continuously administered little by little into the patient's body through the indwelling needle or the like (not shown).

The amount of drug solution administered into the patient's body through the main line 158 per unit time is adjusted by limiting the flow rate of the main line 158 by the flow rate control tube 148. That is, by increasing the length of the flow rate control tube 148, the dose per unit time can be set small, and by decreasing the length of the flow rate control tube 148, the dose per unit time can be set large.

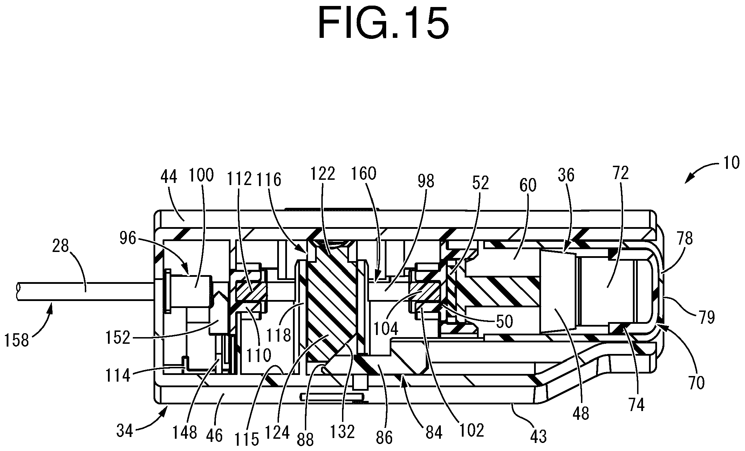

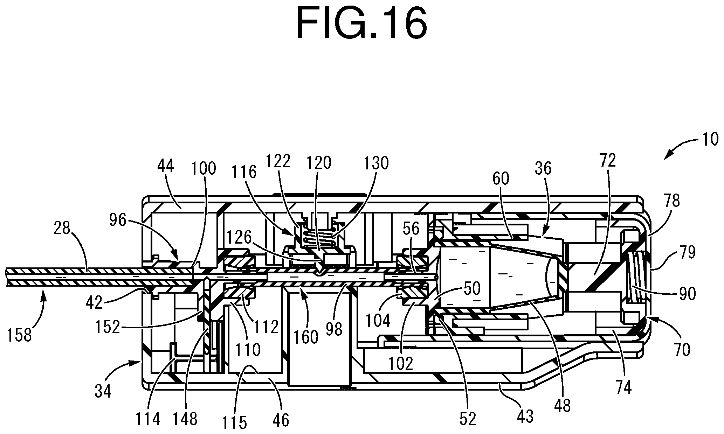

Next, when the patient performs rapid administration to temporarily increase the dose of the drug solution, the patient performs a self-operation of pushing the push button 78 of the drug solution injection controller 10. When the push button 78 is pushed by the patient, the coil spring 90 is compressed between the push button 78 and the plunger 70, as shown in FIGS. 15 and 16. By so doing, the plunger 70 is subjected to a forward urging force based on the elasticity of the compressed coil spring 90. By the push button 78 being pushed to move forward, the engaging hook 64 of the guide member 60 is engaged with the radially inner surface of the hook engaging hole 82 of the push button 78 in the axial direction. Accordingly, the push button 78 is held in a pushed position against the urging force of the coil spring 90. Therefore, the urging force of the coil spring 90 is efficiently applied to the plunger 70.

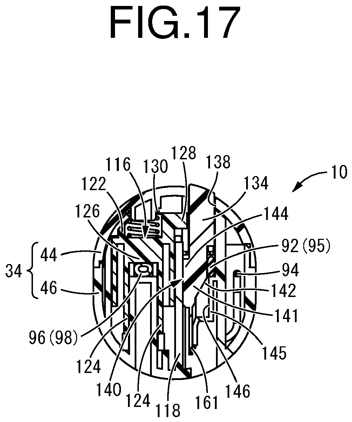

Besides, by the push button 78 being pushed to move forward, the inclined surface 88 of the main drive piece 84 integrally formed with the push button 78 is pressed against the inclined surface 132 of the follower piece 124 integrally formed with the rapid administration valve 116. Then, the forward force exerted on the push button 78 is converted into an upward force by the inclined surfaces 88, 132 and exerted on the rapid administration valve 116. As a result, the rapid administration valve 116 moves upward against the urging force of the coil spring 130, and as shown in FIGS. 16 and 17, the closed state of the outflow side tube 96 by the rapid administration valve 116 is released. It would be acceptable as long as the movement of the main drive piece 84 is changed in direction to the movement of the follower piece 124 and transmitted, and it is not essential that both of the main drive piece 84 and the follower piece 124 are provided with the respective inclined surfaces 88, 132.

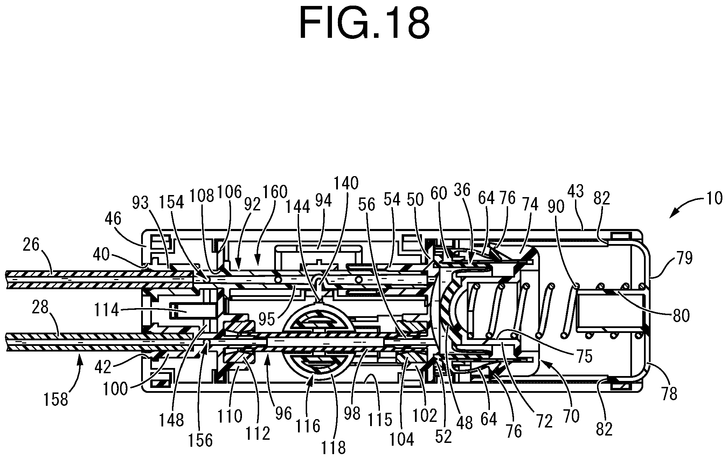

Moreover, with the outflow side tube 96 open, the plunger 70 is pressed against the diaphragm part 48 of the sub-reservoir 36 by the urging force of the coil spring 90, and as shown in FIG. 18, the bottom part of the diaphragm part 48 is pushed forward. As a result, the internal pressure of the sub-reservoir 36 pressed by the plunger 70 increases, and the drug solution stored in the sub-reservoir 36 is injected into the main line 158 via the outflow side tube 96. By so doing, the amount of the drug solution administered into the patient's body from the drug solution administration port 16 is temporarily increased. Here, the inflow side tube 92 is held in a closed state by the closing valve 140, and the lumen of the orifice tube 94 is extremely small in diameter. Thus, when the internal pressure of the sub-reservoir 36 increases, the drug solution in the sub-reservoir 36 is delivered to the outflow side tube 96 without flowing back to the inflow side tube 92.

The drug solution injection controller 10 is configured such that once the push button 78 is pushed, the push button 78 is held at the pushed position. Thus, a single pressing operation administers generally the entire amount of the drug solution stored in the sub-reservoir 36. Therefore, the patient using the drug solution injection controller 10 can rapidly administer a sufficient amount of the drug solution with one push without holding down the push button 78. Regarding the push button 78 pushed by the pressing operation, the spring support part 80 is inserted into the recess 75 of the plunger 70. Therefore, it is possible to sufficiently obtain the stroke of the pressing operation of the push button 78 without increasing the size of the drug solution injection controller 10 in the front-back direction.

Next, when the rapid administration of the drug solution is completed, as shown in FIG. 18, the hook releasing part 76 of the plunger 70 that has moved forward comes into contact with the engaging hook 64 of the guide member 60, and the engaging hook 64 is flexed inward in the left-right direction. By so doing, the engagement of the engaging hook 64 with respect to the radially inner surface of the hook engaging hole 82 of the push button 78 is released, and the push button 78 is allowed to move backward with respect to the housing 34. Further, by the push button 78 being allowed to move backward and moving backward based on the elasticity of the coil spring 90, the urging force of the coil spring 90 acting on the plunger 70 is canceled.

Moreover, when the push button 78 moves backward, the contact between the main drive piece 84 integrally formed with the push button 78 and the follower piece 124 integrally formed with the rapid administration valve 116 is released. By so doing, the clamp protrusion 126 of the rapid administration valve 116 is pressed against the outflow side tube 96 by the urging force of the coil spring 130, and the outflow side tube 96 is switched to the closed state by the rapid administration valve 116.

Furthermore, a part of the drug solution delivered from the main reservoir 22 to the upstream side external line 26 fills the sub-reservoir 36 through the inflow side tube 92 and the orifice tube 94. By so doing, the drug solution in the sub-reservoir 36, which has been reduced by rapid administration, gradually increases. The plunger 70 that pressed the diaphragm part 48 of the sub-reservoir 36 during rapid administration does not significantly hinder the deformation of the diaphragm part 48 because the urging force of the coil spring 90 has been released. Thus, filling the sub-reservoir 36 with the drug solution accompanied by the deformation of the diaphragm part 48 will be permitted.

Additionally, as the amount of the drug solution in the sub-reservoir 36 increases, the plunger 70 is pushed by the diaphragm part 48 and moves backward. Then, the plunger 70 is moved to the initial position by the sub-reservoir 36 being filled with generally the same amount of the drug solution as before the rapid administration. Further, due to the plunger 70 moving backward, the push button 78 is moved backward by the urging force of the coil spring 90, and the push button 78 is moved to the initial position in the same manner as the plunger 70.

Since a part of the flow path for supplying the drug solution to the sub-reservoir 36 is constituted by the orifice tube 94, the amount of the drug solution accumulated in the sub-reservoir 36 is limited after the rapid administration of the drug solution, and a predetermined time is required for filling the sub-reservoir 36 with the drug solution to an administrable amount. With this configuration, rapid administration of the drug solution is prevented from being repeatedly executed in a short time, thereby preventing overdose of the drug solution. The filling time until the rapid administration of the drug solution becomes possible again can be set by the flow rate of the orifice tube 94. That is, when the flow rate of the drug solution flowing through the orifice tube 94 is small, it takes time for the drug solution to accumulate in the sub-reservoir 36, so that the time interval for rapid administration becomes long. Further, the flow rate of the orifice tube 94 can be adjusted by adjusting the inner diameter and length of the orifice tube 94. Generally, the smaller the inner diameter is set, the smaller the flow rate becomes, and the longer the length is set, the smaller the flow rate becomes.

Moreover, in the present practical embodiment, as shown in FIG. 4, the peripheral wall of the housing 34 is provided with a slit 162 extending in the axial direction, and by a protrusion-shaped mark 164 provided to the plunger 70 being inserted into the slit 162, the relative position of the plunger 70 with respect to the housing 34 in the axial direction can be recognized from the outside. That is, the state where the mark 164 has moved to the vicinity of the back end of the slit 162 indicates the state where the sub-reservoir 36 is filled with the drug solution.

The drug solution injection controller 10 having a structure according to the present practical embodiment is easily provided in the drug solution administration device 12 by the upstream side external line 26 constituting the main line 158 being connected to the inflow side port 93, while the downstream side external line 28 being connected to the outflow side port 100.

Besides, the flow rate control tube 148 serving as the flow rate control part constituting the main line 158 is provided to the drug solution injection controller 10, and the drug solution injection controller 10 has a structure including a part of the main line 158 and the entire sub-line 160. Therefore, by adopting the drug solution injection controller 10, the sub-line 160 and the flow rate control tube 148 can be easily provided in the drug solution administration device 12.

Furthermore, since the flow rate control tube 148 is provided in the drug solution injection controller 10, the configuration of the upstream side external line 26 can be simpler than that in the case where they are provided in the middle of the upstream side external line 26. In particular, in the present practical embodiment, the drug solution injection controller 10 includes the rigid housing 34, and the flow rate control tube 148 is incorporated in the housing in a housed state. Therefore, as compared with the case where the flow rate control tube 148 is provided so as to be exposed to the outside, the handling of the upstream side external line 26 becomes easier, and the flow rate control tube 148 is protected by the housing 34.

Additionally, since the flow rate control part is constituted by the small-diameter flow rate control tube 148, the flow rate control part that adjusts the flow rate of the main line 158 is realized by a simple structure. Moreover, there is provided the latching protrusion 114 around which the flow rate control tube 148 can be wound to be held. Thus, for example, when the flow rate control tube 148 is long, by the flow rate control tube 148 being wound around the latching protrusion 114, the flow rate control tube 148 can be stably held and prevented from interfering with other members or the like.

Moreover, in the present practical embodiment, the branch point 154 and the confluence point 156 of the main line 158 and the sub-line 160 are provided in the drug solution injection controller 10. Thus, in comparison with the case where the branch point 154 and the confluence point 156 are provided in the middle of the external lines 26, 28, the configuration of the external lines 26, 28 can be simplified. In particular, in the present practical embodiment, the branch point 154 and the confluence point 156 are incorporated in the housing 34 in a housed state. Thus, in comparison with the case where the branch point 154 and the confluence point 156 are provided so as to be exposed to the outside, the external lines 26, 28 can be easily handled, and it is also possible to prevent an unintended force from being applied to the branch point 154 and the confluence point 156 from the outside.

Besides, the priming flow path 95 extends straightly in the front-back direction, and the orifice tube 94 provided in parallel with the priming flow path 95 is provided below the priming flow path 95. With this configuration, the priming flow path 95 having a larger diameter than that of the orifice tube 94 can be provided with a simple structure, and the housing 34 can also be prevented from being increased in diameter due to the large-diameter priming flow path 95 extending to the lateral side. Further, even if the orifice tube 94 is arranged on the lateral side (to the lower side) of the priming flow path 95, since the orifice tube 94 has a small diameter, a problem of increase in size of the housing 34 is unlikely to occur. Moreover, since the orifice tube 94 has a small diameter and is flexible, it is easy to arrange the orifice tube 94 so as to extend on a curved path.

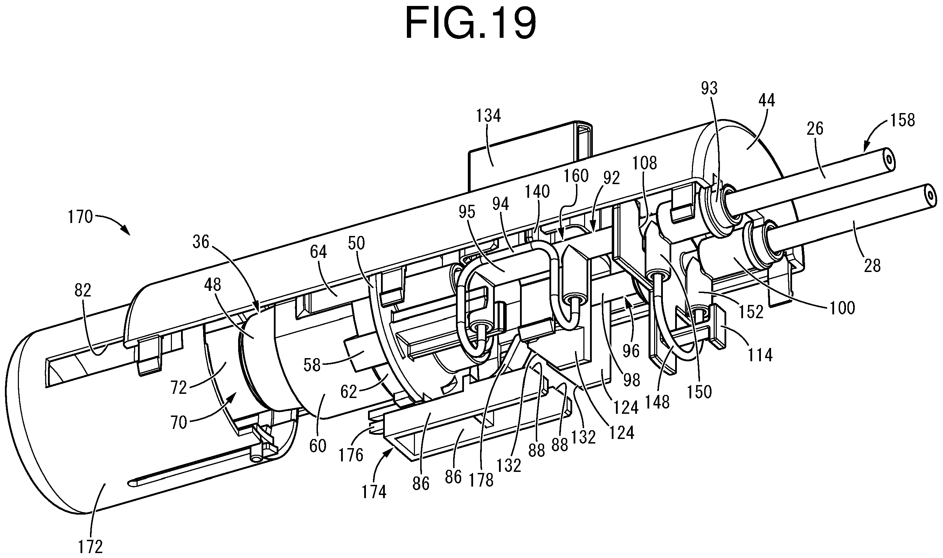

FIG. 19 shows a drug solution injection controller 170 according to a second practical embodiment of the present invention, with the lower half portion (46) of the housing 34 removed. In the following description, the members and parts substantially the same as those in the first practical embodiment are designated by the same reference numerals in the drawings, and the description thereof will be omitted.

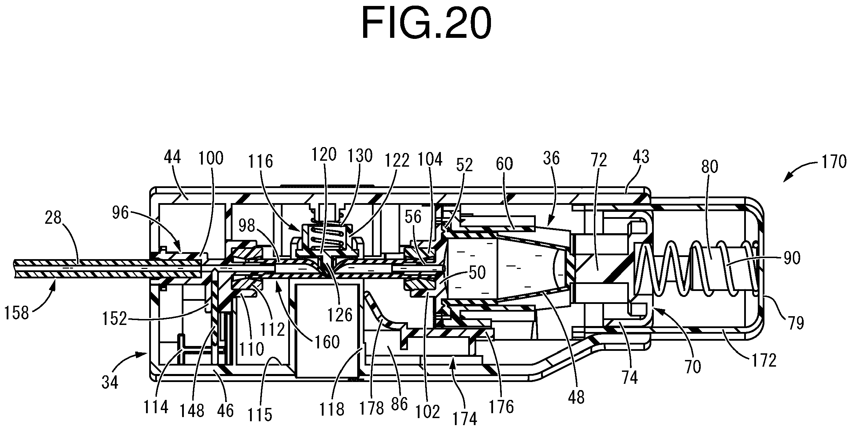

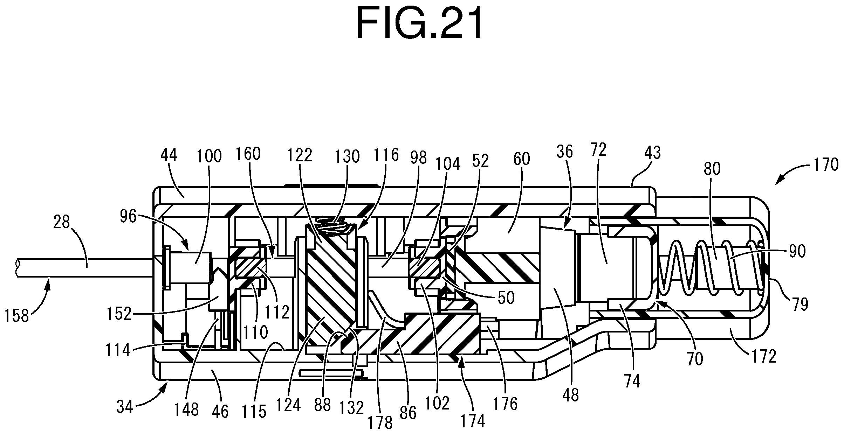

As shown in FIGS. 20 and 21, the drug solution injection controller 170 includes a push button 172 serving as a pressing operation member protruding backward of the housing 34 (to the right in FIG. 20). As shown in FIGS. 19 and 21, the push button 172 does not include the main drive piece 84 as compared with the push button 78 of the first practical embodiment, and has a generally round tubular shape with a bottom.

Besides, as shown in FIGS. 19 and 21, a main drive member 174 is arranged in the lower part of the housing 34. The main drive member 174 has a structure in which two sliding contact parts 86, 86 arranged in parallel are connected to each other at the proximal end portion (the back end portion). The distal end surfaces (the front end faces) of the sliding contact parts 86, 86 each have the inclined surface 88 that inclines downward toward the distal end side. Further, the main drive member 174 includes a contact projection 176 that projects backward. Moreover, the main drive member 174 includes an elastic piece 178 extending forward. The elastic piece 178 has a plate shape, curves in the thickness direction and inclines upward as it goes forward, while being elastically deformable in the thickness direction.

The main drive member 174 is movable back and forth along the inner surface of the lower wall of the housing 34 in the front-back direction. In the injection standby state of the main drive member 174 shown in FIGS. 20 and 21 in which the push button 172 is not pushed, the sliding contact parts 86, 86 are remote backward from the follower piece 124 that is integrally formed with the rapid administration valve 116, while the contact projection 176 is remote forward from the push button 172.

Besides, in the said injection standby state, the elastic piece 178 is in contact with the valve guide part 118 of the housing 34, and the forward displacement of the main drive member 174 is limited by the elastic piece 178. Further, in the present practical embodiment, a step 180 is formed on the inner surface of the lower wall of the housing 34, and the backward displacement of the main drive member 174 is limited by the contact with the step 180.

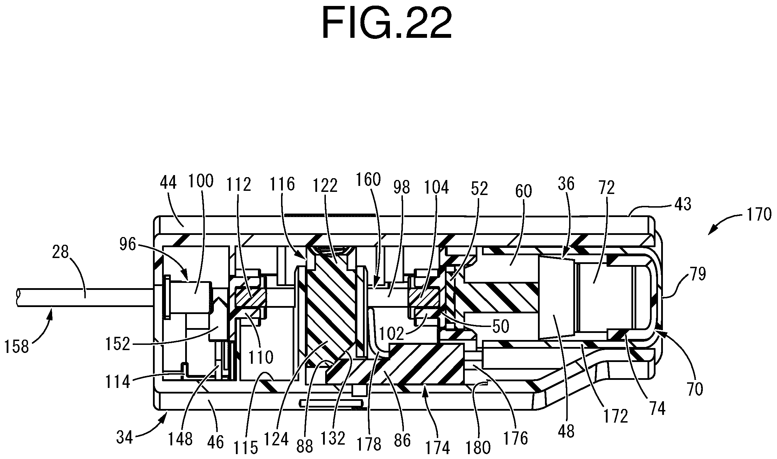

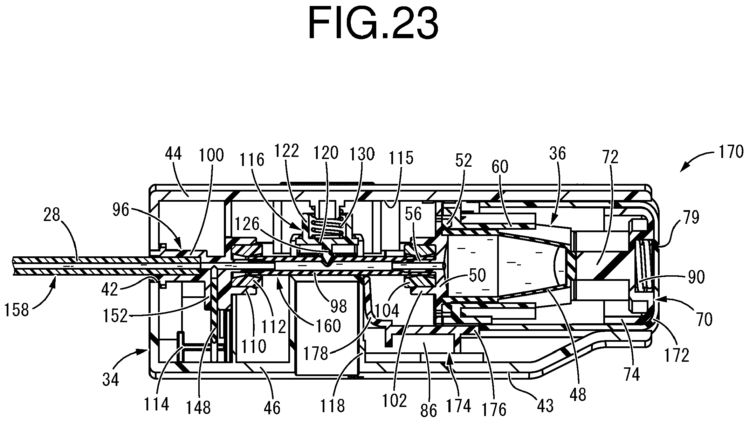

When the push button 172 is pushed by the user, as shown in FIG. 22, the front end part of the push button 172 moved forward comes into contact with the contact projection 176 of the main drive member 174, and the main drive member 174 is pushed forward by the push button 172. The pushed main drive member 174 moves forward against the elastic force of the elastic piece 178, and the inclined surfaces 88, 88 of the sliding contact parts 86, 86 are pressed against the inclined surfaces 132, 132 of the follower pieces 124, 124. By so doing, the follower pieces 124, 124 are pushed upward, and the rapid administration valve 116 integrally formed with the follower pieces 124, 124 is pushed upward. As a result, as shown in FIG. 23, the connection tube 98 that has been crushed and closed by the rapid administration valve 116 is switched to the open state due to the displacement of the rapid administration valve 116, and the drug solution stored in the sub-reservoir 36 is rapidly administered through the connection tube 98.

After the rapid administration of the drug solution is completed, the main drive member 174 moves backward based on the elasticity of the elastic piece 178 as the push button 172 moves backward due to the urging force of the coil spring 90. By so doing, the rapid administration valve 116 moves downward due to the urging force of the coil spring 130, and the rapid administration valve 116 closes the connection tube 98 again. The main drive member 174 that moves backward may come into contact with the step 180 of the housing 34, or may stop at a position away from the step 180 forward. Further, if the main drive member 174 is configured to be pushed backward to move by utilizing the movement of the rapid administration valve 116 due to the urging force of the coil spring 130, the elastic piece 178 may be obviated.

As in the present practical embodiment, the main drive member 174 including the sliding contact parts 86, 86 is separated from the push button 172, so that the push button 172 and the main drive member 174 can be attached to the housing 34 separately. This facilitates the work of attaching the push button 172 and the main drive member 174 to the housing 34. Further, the push button 172 and the main drive member 174 are remote from each other in the injection standby state. Thus, in comparison with the structure in which the push button 78 and the main drive piece 84 are integrally connected as in the first practical embodiment, the dimensional error of the components can be largely allowed, so that the defective assembly of the push button 172 and the main drive member 174 is less likely to occur.

While the present invention has been described in detail hereinabove in terms of the practical embodiments, the invention is not limited by the specific disclosures thereof. For example, the flow rate control part is not limited to a shape of a small-diameter tube like the flow rate control tube 148 shown in the preceding practical embodiment, but may have, for example, a structure in which plate members are overlapped so that the flow rate of the drug solution is adjusted by a narrow flow path formed between the overlapped plate members, or the like. As for the limiting flow path constituted by the orifice tube 94 in the preceding practical embodiment as well, a different structure can be adopted similarly to the flow rate control part.

In the preceding practical embodiment, the structure in which the flow rate control tube 148 is incorporated in the housing 34 is illustrated. However, it would also be possible that, for example, the inflow side port 93 and the outflow side port 100 are extended to the front of the housing 34, and the flow rate control tube 148 connecting the inflow side port 93 and the outflow side port 100 is provided on the front outer side of the housing 34. In this case, since the branch point 154 and the confluence point 156 of the main line 158 and the sub-line 160 are located outside the housing 34, the entire main line 158 is provided outside the housing 34, and a part of the sub-lines 160 is provided on the outside of the housing 34. In this way, the flow rate control tube 148 serving as the flow rate control part can be arranged outside the flow path arrangement space 115 in the housing 34. That is, the expression that a component is provided to the drug solution injection controller 10 does not necessarily mean the only case where the component is incorporated in the housing 34 of the drug solution injection controller 10 in a housed state.

Moreover, in the case where the flow rate control part is provided outside the housing 34, the confluence point 156 of the main line 158 and the sub-line 160 can be provided inside the housing 34. In this case, the external flow path on the inflow side of the main line 158, the external flow path on the inflow side of the sub-line 160, and the external flow path on the outflow side of the main line 158 are connected to the drug solution injection controller.

Besides, in the preceding practical embodiment, described is a structure in which the opposite ends of the flow rate control tube 148 are inserted up to the proximal ends of the connecting parts 150, 152, and are connected to the branch point 154 and the confluence point 156 of the main line 158 and the sub-line 160. However, for example, the flow rate control tube 148 may be inserted into the opening parts so as not to reach the proximal ends of the connecting parts 150, 152, and may indirectly connect the branch point 154 and the confluence point 156 via the connecting parts 150, 152.

In the preceding practical embodiment, the upstream side external line 26 is inserted into and connected to the inflow side port 93 of the inflow side tube 92, while the downstream side external line 28 is inserted into and connected to the outflow side port 100 of the outflow side tube 96, so that the external lines 26, 28, the inflow side tube 92, and the outflow side tube 96 are independent of one another. However, it is also possible that the upstream side external line 26 and the inflow side tube 92 are constituted by a continuous integrated tube, and it is also possible that the downstream side external line 28 and the outflow side tube 96 are constituted by a continuous integrated tube. As can be seen from this, the inflow side port 93 and the outflow side port 100 are not necessarily limited to the structure in which independent lines are inserted, but refer to the opposite ends of the portion of the main line 158 provided in the drug solution injection controller 10.

Additionally, in the preceding practical embodiment, the inflow side tube 92 and the outflow side tube 96 are arranged side by side in the left-right direction that is orthogonal to the major axis direction in the cross section of the housing 34. However, the inflow side tube 92 and the outflow side tube 96 may be arranged side by side, for example, in the major axis direction (the vertical direction) in the cross section of the housing 34. Needless to say, the inflow side port 93 and the outflow side port 100 can also be arranged side by side in the major axis direction in the cross section of the housing 34. Further, since the external lines 26, 28 extend forward from the front wall part located on the opposite side of the pressing operation surface 79 in the housing 34, the complication of the flow path in the housing 34 is prevented. However, it would also be acceptable that a member for preventing kinking is added inside or the like, so that the external lines 26, 28 can be extended from the pressing operation surface 79 side (the back side) of the housing 34.

### KEYS TO SYMBOLS

10, 170: drug solution injection controller, 12: drug solution administration device, 16: drug solution administration port, 22: main reservoir, 34: housing, 36: sub-reservoir, 43: gripping surface, 70: plunger, 75: recess, 78, 172: push button (pressing operation member), 79: pressing operation surface (fingertip pressing surface), 80: spring support part (protrusion), 90: coil spring, 92: inflow side tube (inflow side flow path), 93: inflow side port (port for connecting external flow path), 94: orifice tube (limiting flow path), 95: priming flow path, 96: outflow side tube (outflow side flow path), 100: outflow side port (port for connecting external flow path), 114: latching protrusion (regulating part), 115: flow path arrangement space, 116: rapid administration valve, 140: closing valve, 141: valve body, 142: inclined contact side, 148: flow rate control tube (flow rate control part, connecting flow path), 158: main line, 160: sub-line

## Claims

1. A drug solution injection controller comprising:
a housing;
a sub-reservoir housed by the housing; and
a pressing operation member for performing rapid administration of a drug solution, the pressing operation member being attached to the housing so as to be movable back and forth, wherein
a pressing operation surface of the pressing operation member, the sub-reservoir, and a flow path arrangement space in which an inflow side flow path and an outflow side flow path of the drug solution with respect to the sub-reservoir are arranged are provided side by side in series in a direction of movement of the pressing operation member, and
the flow path arrangement space is provided with a rapid administration valve for opening and closing the outflow side flow path, a limiting flow path provided on the inflow side flow path, a priming flow path bypassing the limiting flow path on the inflow side flow path, and a closing valve for closing the priming flow path.

2. The drug solution injection controller according to claim 1, wherein the sub-reservoir is arranged closer to the pressing operation member than a center of the housing in the direction of movement of the pressing operation member.

3. The drug solution injection controller according to claim 1 or 2, further comprising:
a plunger for pressing the sub-reservoir during pressing operation of the pressing operation member; and
a coil spring urging the plunger toward the sub-reservoir, wherein
the plunger includes a recess opening toward the pressing operation member, and the pressing operation member includes a protrusion protruding toward the recess, and
opposite ends of the coil spring are positioned by the recess of the plunger and the protrusion of the pressing operation member.

4. The drug solution injection controller according to any one of claims 1-3, wherein the outflow side flow path is configured to be closed by being pressed by the rapid administration valve in a direction orthogonal to the direction of movement of the pressing operation member.

5. The drug solution injection controller according to any one of claims 1-4, wherein the priming flow path is configured to be closed by being pressed by the closing valve in a direction orthogonal to the direction of movement of the pressing operation member.

6. The drug solution injection controller according to any one of claims 1-5, wherein the priming flow path extends straightly from the sub-reservoir in the direction of movement of the pressing operation member.

7. The drug solution injection controller according to any one of claims 1-6, wherein
the housing has an elongated shape, and an outer peripheral surface of the housing comprises a gripping surface to be gripped by a hand of a user,
the pressing operating member is provided on one side in a longitudinal direction of the housing, and is movable back and forth in the longitudinal direction of the housing, and
the pressing operation surface serves as a fingertip pressing surface that the user presses with a thumb on a distal end surface of the pressing operation member.

8. The drug solution injection controller according to any one of claims 1-7, wherein a cross-sectional shape of the housing in a direction orthogonal to the direction of movement of the pressing operation member is a flat hollow cross-sectional shape.

9. The drug solution injection controller according to any one of claims 1-8, wherein the closing valve has a plate-shaped valve body including an inclined contact side for closing the priming flow path so as to crush the priming flow path.

10. The drug solution injection controller according to any one of claims 1-9, further comprising a flow rate control part connecting the inflow side flow path and the outflow side flow path so as to continuously administer the drug solution without passing through the sub-reservoir.

11. The drug solution injection controller according to claim 10, wherein the flow rate control part is provided at a position farther from the pressing operation member than the limiting flow path, the priming flow path, and the closing valve are.

12. The drug solution injection controller according to claim 10 or 11, wherein
a cross-sectional shape of the housing in a direction orthogonal to the direction of movement of the pressing operation member is a flat hollow cross-sectional shape,
a connecting flow path is provided so as to connect the inflow side flow path and the outflow side flow path while including the flow rate control part, and
at least one of a direction of extension of the connecting flow path, a direction of movement of the rapid administration valve with respect to the outflow side flow path, and a direction of movement of the closing valve with respect to the priming flow path coincides with a major axis direction of a cross section of the housing.

13. The drug solution injection controller according to any one of claims 10-12, wherein the inflow side flow path and the outflow side flow path include respective ports for connecting external flow paths, and a connecting flow path including the flow rate control part is provided so as to connect the ports.

14. The drug solution injection controller according to claim 13, wherein the connecting flow path is arranged in the housing so as to curve toward a circumferential direction of the housing.

15. The drug solution injection controller according to any one of claims 10-14, further comprising a regulating part for regulating movement of the flow rate control part.

16. The drug solution injection controller according to any one of claims 10-15, wherein the flow rate control part is provided in the housing in a housed state.
